# EUROPEAN PATENT APPLICATION

(11) **EP 4 674 864 A1**
(43) Date of publication of application: **07.01.2026**
(21) Application number: 25770389.2
(22) Date of filing: 23.04.2025
(51) Int. Cl.: C07K 14/405, C12N 15/11, C12N 15/864, A61K 38/16, A61P 27/02

(54) **NEW CHANNELRHODOPSIN VR3.0 AND APPLICATION THEREOF**

(30) Priority: 03.09.2024 CN 202411232798
(71) Applicant: ZHONGMOU THERAPEUTICS CO., LTD., Wuhan, Hubei 430074 (CN)
(72) Inventor: SHEN, Yin, Wuhan, Hubei 430074 (CN); GAO, Shiqiang, Wuhan, Hubei 430074 (CN)
(74) Representative: Lavoix
(86) International application number: PCT/CN2025/090714
(87) International publication number: WO 2025/214507

(57) **Abstract**

The application provides a novel light-activated channel protein VR3.0 and uses thereof. The light-activated channel protein VR3.0 series provided in this application has a wide range of photosensitive wavelengths, higher sensitivity to light response, faster photoresponse kinetics, maintaining stable photocurrent signals under high-frequency stimulation while exhibiting good response amplitude and frequency under light stimulation of various wavelengths (especially white light, natural light). The light-activated channel protein VR3.0 series provided in this application has a clear therapeutic effect on retinal photoreceptor cell degenerative diseases, and can be used to prepare drugs for restoring the photoreceptor function of the retina, restoring the visual or photosensitive ability of subjects, and treating retinal degenerative diseases.

## Description

### TECHNICAL FIELD

This application belongs to the field of biomedicine, specifically, it relates to a novel light-activated channel protein VR3.0 and uses thereof.

### BACKGROUND

Retinal photoreceptor cell degenerative disease is a type of degenerative disease primarily characterized by progressive functional loss of photoreceptor cells and retinal pigment epithelial cells. This type of disease is mainly caused by genetic mutations or dysfunction of retinal pigment epithelial cells (RPE cells), with typical examples including retinitis pigmentosa (RP) and age-related macular degeneration (AMD), both of which are major and intractable blinding eye diseases. The prevalence of hereditary photoreceptor degenerative disorder is about 1/3500-1/4000. Currently, there are 400000 patients with retinitis pigmentosa in China and over 1.5 million patients worldwide. The prevalence of secondary retinal photoreceptor degenerative diseases caused by factors such as drug administration and diseases is also increasing.

Due to the irreversible apoptosis of photoreceptor cells in retinal photoreceptor cell degenerative disease and the high genetic heterogeneity of most such diseases, the treatment of related diseases has become extremely challenging. Current therapeutic approaches primarily include stem cell transplantation, gene therapy, retinal prosthesis implantation, and optogenetic therapy. Among them, optogenetic therapy leverages the structural integrity of residual cells in retinal degenerative diseases by using recombinant adeno-associated virus (AAV) as a vector to target the expression of light-activated channel proteins in cone cells for early stage of degenerative diseases, bipolar cells, or ganglion cells for mid-to-late stage degenerative diseases, thereby restoring retinal photosensitivity. In addition, stem cell transplantation, virtual reality systems, and holographic imaging technology can be combined to restore visual function.

Optogenetics-based therapy, as an ophthalmic treatment strategy that is not reliant on the correction of specific gene mutations and enables light responsiveness at the single-cell level, has great potential for the treatment of retinal photoreceptor degenerative disorders. The photosensitive proteins employed in optogenetic visual restoration strategies are mainly divided into two categories: microbial photosensitive proteins and mammalian photosensitive proteins. Microbial photosensitive proteins typically exhibit fast kinetic, but low photosensitivity, such as the cation-permeable photosensitive protein Channelrhodopsin-2 (ChR2), which was the first photosensitive protein applied for visual function restoration. Endogenous mammalian photosensitive proteins belong to the G Protein Coupled Receptors (GPCRs) family, with seven transmembrane alpha-helix domains. They typically exhibit high photosensitivity but lack sufficient kinetic performance, such as rhodopsin derived from rod photoreceptor cells (Rod). Currently, available optogenetic tools (i.e., photosensitive proteins) used for visual restoration fails to adequately meet the dual requirements of high light sensitivity and fast photoresponse kinetics. They also present issues such as ion selectivity discrepancies and side effects like intracellular acidification. Therefore, it is necessary to develop a class of light-activated channel proteins with enhanced light sensitivity, faster photoresponse kinetics, minimal side effects, and the ability to maintain stable current signals under high-frequency light stimulation. Coupled with more efficient gene delivery vectors targeting retinal cells, optogenetic therapy can be better used in the treatment of retinal photoreceptor cell degenerative diseases.

### SUMMARY

To address the limitations of existing technology, this application provides a novel light-activated channel protein VR3.0 and uses thereof.

Specifically, this application involves the following aspects:
1. A light-activated channel protein comprising a channelrhodopsin comprising an amino acid sequence shown in SEQ ID NO.1 or a variant thereof, wherein the variant is selected from any one of the following:
   (1) a protein obtained by truncating 1-23 amino acids at the N-terminus of the channelrhodopsin;
   (2) a protein obtained by truncating 90-189 amino acids at the C-terminus of the channelrhodopsin;
   (3) a protein obtained by truncating 1-23 amino acids at the N-terminus and 90-189 amino acids at the C-terminus of the channelrhodopsin; or
   (4) a protein having more than 90%, 91%, 92%, 93%, 94%, 95%, 96%, 97%, 98%, or 99% sequence identity with the protein described in (1), (2), or (3).
2. The light-activated channel protein according to item 1, wherein the amino acid sequence of the channelrhodopsin is shown in SEQ ID NO.1.
3. The light-activated channel protein according to item 1 or 2, wherein the variant is a protein obtained by truncating 5-15 amino acids at the N-terminus of the channelrhodopsin.
4. The light-activated channel protein according to item 1 or 2, wherein the variant is a protein obtained by truncating 102-179 amino acids at the C-terminus of the channelrhodopsin.
5. The light-activated channel protein according to item 1 or 2, wherein the variant is a protein obtained by truncating 5-15 amino acids at the N-terminus and 102-179 amino acids at the C-terminus of the channelrhodopsin.
6. The light-activated channel protein according to any one of items 1-5, wherein the amino acid sequence of the variant is shown in any one of SEQ ID NO.8-26, or
   has more than 90%, 91%, 92%, 93%, 94%, 95%, 96%, 97%, 98%, or 99% sequence identity with any one of SEQ ID NO.8-26.
7. The light-activated channel protein according to any one of items 1-6, wherein the light-activated channel protein further comprises an LR signal peptide fused to the N-terminus of the channelrhodopsin or a variant thereof,
   and/or a T peptide linked to the C-terminus of the channelrhodopsin or a variant thereof to enhance cell membrane expression efficiency, and an endoplasmic reticulum output signal sequence E peptide linked to the T peptide.
8. The light-activated channel protein according to item 7, wherein the amino acid sequence of the LR signal peptide is shown in SEQ ID NO.2, the amino acid sequence of the T peptide is shown in SEQ ID NO.4, and the amino acid sequence of the E peptide is shown in SEQ ID NO.5.
9. The light-activated channel protein according to item 7 or 8, wherein a linker peptide sequence is further included between any two of the channelrhodopsin or a variant thereof, the T peptide, and the E peptide.
10. The light-activated channel protein according to item 9, wherein the amino acid sequence of the linker peptide is shown in SEQ ID NO.28.
11. A nucleic acid molecule comprising a nucleotide sequence encoding the light-activated channel protein according to any one of items 1-10.
12. A vector comprising the nucleic acid molecule according to item 11.
13. A recombinant virus comprising the nucleic acid molecule according to item 11 or the vector according to item 12.
14. The recombinant virus according to item 13, wherein the recombinant virus is a recombinant adeno-associated virus.
15. A pharmaceutical composition comprising the light-activated channel protein according to any one of items 1-10, the nucleic acid molecule according to item 11, the vector according to item 12, or the recombinant virus according to item 13 or 14, and a pharmaceutically acceptable carrier.
16. Use of the pharmaceutical composition according to item 15 in the preparation of a medicament for treating retinal photoreceptor cell degenerative diseases.
17. The use according to item 16, wherein the retinal photoreceptor cell degenerative disease comprises retinitis pigmentosa (RP), age-related macular degeneration (AMD), cone-rod dystrophy, and Leber congenital amaurosis (LCA).
18. A method for treating retinal photoreceptor cell degenerative diseases, comprising administering a therapeutic effective amount of the pharmaceutical composition according to item 15 to a subject.
19. The method according to item 18, wherein the retinal photoreceptor cell degenerative disease comprises retinitis pigmentosa (RP), age-related macular degeneration (AMD), cone-rod dystrophy, and Leber congenital amaurosis (LCA).

Advantages and beneficial effects of this application:
The light-activated channel protein VR3.0 series provided in this application has a wide range of photosensitive wavelengths, higher sensitivity to light response, faster photoresponse kinetics, maintaining stable photocurrent signals under high-frequency stimulation while exhibiting good response amplitude and frequency under light stimulation of various wavelengths (especially white light, natural light). The light-activated channel protein VR3.0 series provided in this application has a clear therapeutic effect on retinal photoreceptor cell degenerative diseases, and can be used to prepare drugs for restoring the photoreceptor function of the retina, restoring the visual or photosensitive ability of subjects, and treating retinal degenerative diseases.

### BRIEF DESCRIPTION OF THE DRAWINGS

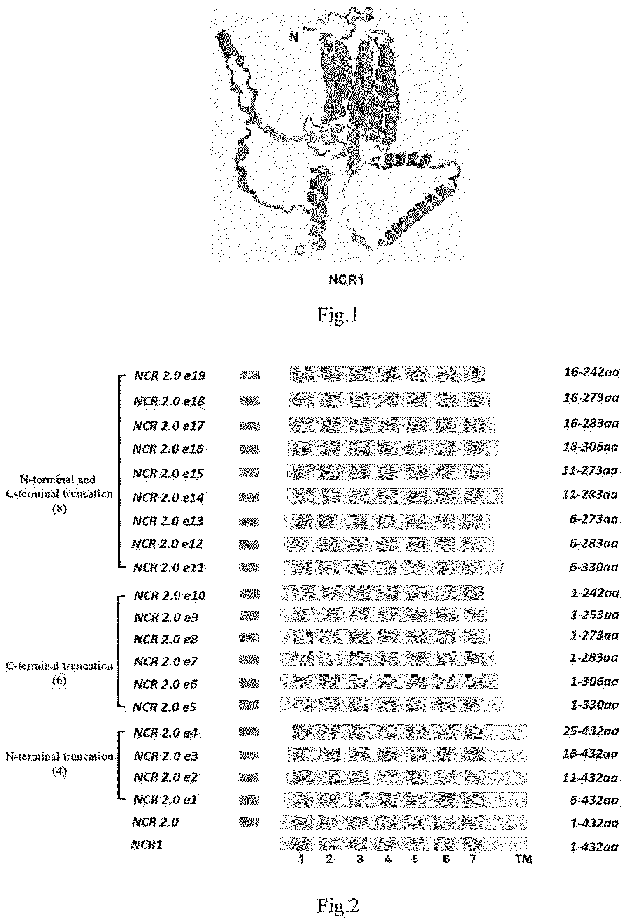

**Fig. 1** shows the simulated three-dimensional structural diagram of the light-activated protein NCR1 (1-432aa).
**Fig. 2** shows a schematic diagram of the amino acid structure of the light-activated protein VR3.0 series modified from the light-activated protein NCR1.

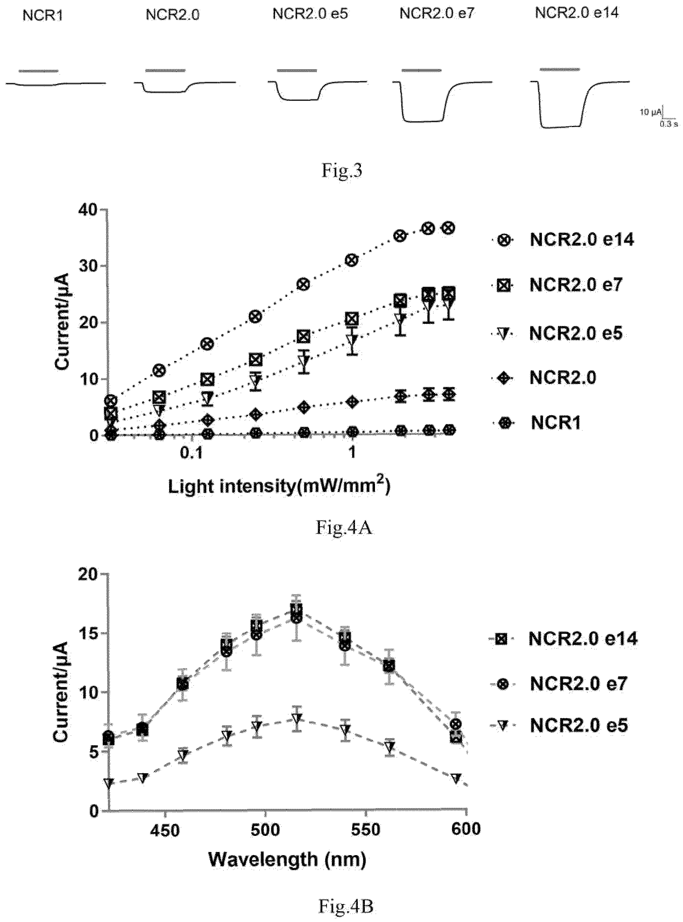

**Fig. 3** shows the waveform of photoresponse electrophysiological signals of some mutants of the light-activated protein VR3.0 series at the *Xenopus laevis* oocyte level.
**Fig. 4A** shows the current response trends of some variants of the light-activated protein VR3.0 series expressed in *Xenopus laevis* oocytes under increasing light intensities; **Fig. 4B** shows the photocurrent levels produced by some variants of the light-activated protein VR3.0 series in *Xenopus laevis* oocytes in response to light wavelengths ranging from 420 nm to 600 nm.

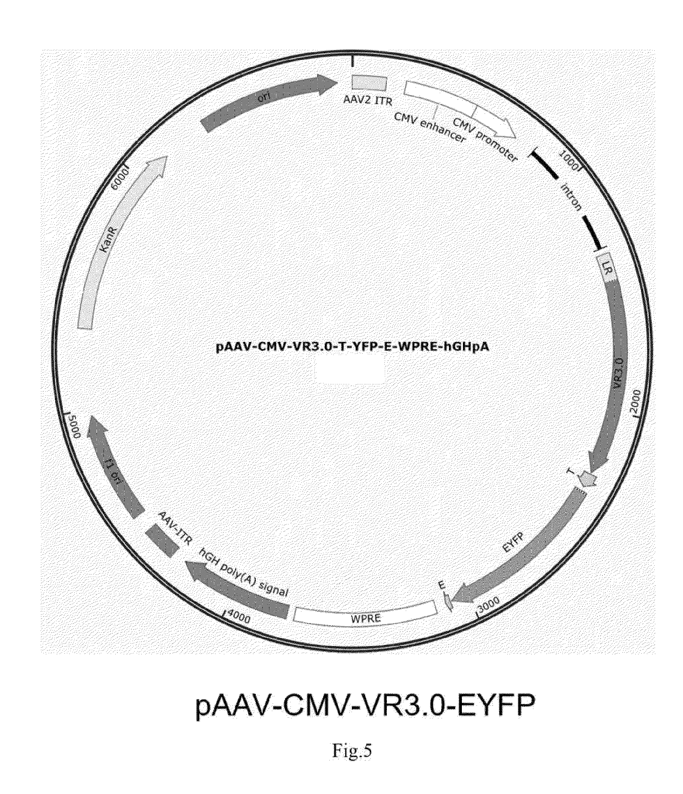

**Fig. 5** is a schematic diagram of the core viral vector carrying the expression cassette for the light-activated protein VR3.0 series.

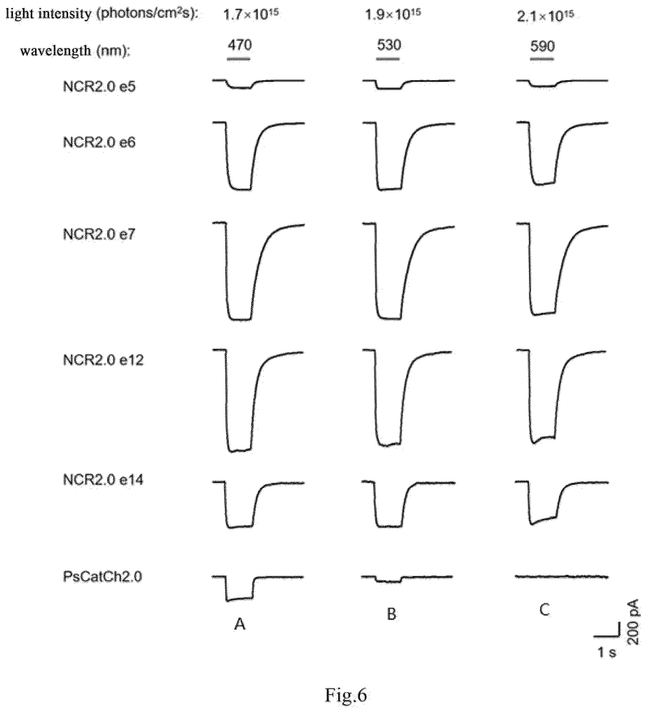

**Fig. 6** shows after expressing some variants of the light-activated protein VR3.0 series and the control group PsCatCh2.0 at the HEK293T cell level, A. comparison of photoresponse current plots under 470 nm wavelength light with an intensity of 1.7×10¹⁵ photons/cm²s; B. comparison of photoresponse current plots under 530 nm wavelength light with an intensity of 1.9×10¹⁵ photons/cm²s; C. comparison of photoresponse current plots under 590 nm wavelength light with an intensity of 2.1 ×10¹⁵ photons/cm²s.

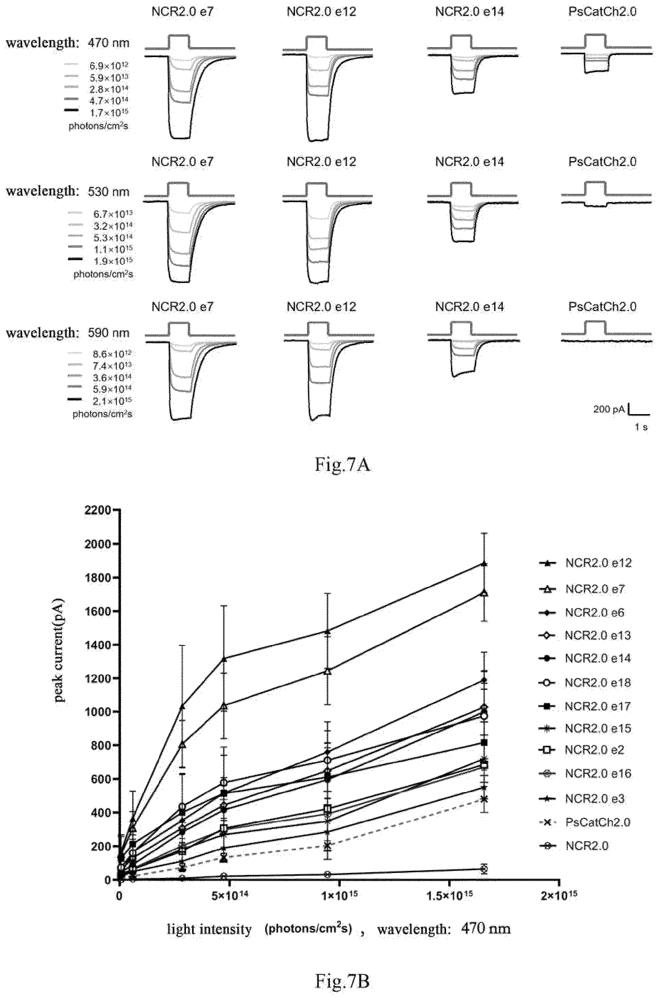

**Fig. 7** shows after expressing some variants of the light-activated protein VR3.0 series in HEK293T cells, the photoresponse current of different variants of the light-activated channel protein VR3.0 under different wavelengths (470 nm, 530 nm, 590 nm) and varying light intensities **(****Fig. 7A****);** as well as the photocurrent response curves of different light-activated protein VR3.0 variants under 470 nm wavelength at different light intensities **(****Fig. 7B****).**

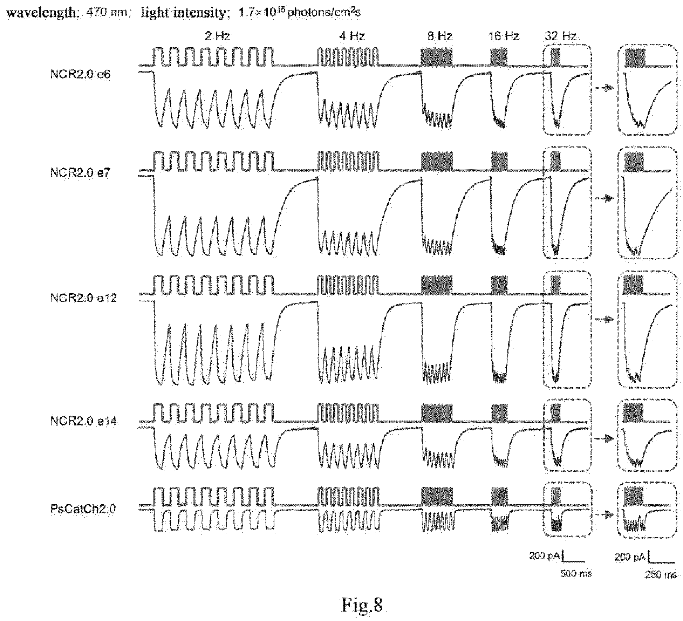

**Fig. 8** shows after expressing some variants of the light-activated channel protein VR3.0 series and the control group PsCatCh2.0 in HEK293T cells, the photoresponse current generated at stimulation frequencies of 2 Hz, 4 Hz, 8 Hz, 16 Hz, and 32 Hz under 470nm wavelength light with an intensity of 1.7×10¹⁵ photons/cm²s.

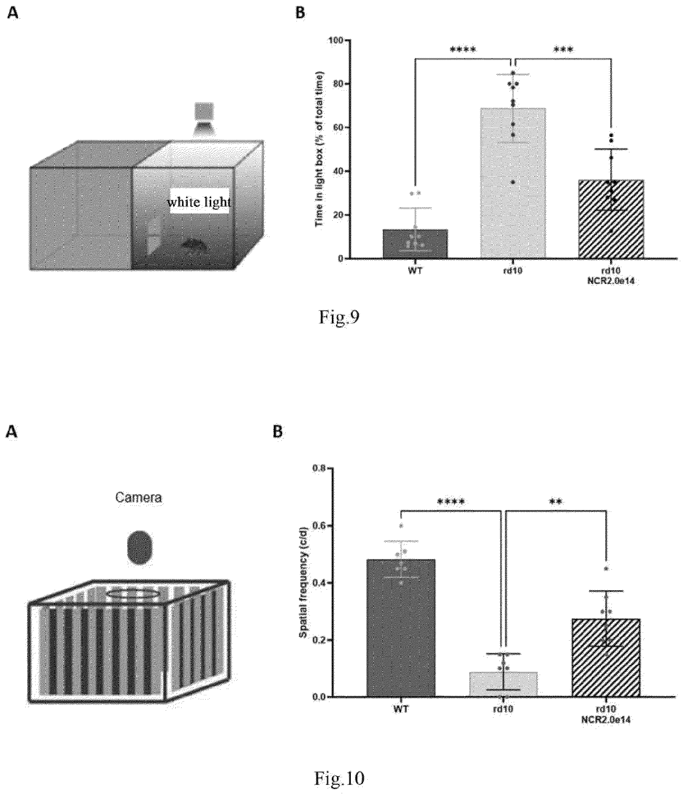

**Fig. 9** shows the light-avoidance responses in the light-dark box test for C57BL/6J mice, *rd10* mice treated with intravitreal injection of rAAV2-CMV-VR3.0-EYFP, and untreated littermate *rd10* mice. A is the schematic diagram of the light-dark box experiment; B is the statistical bar chart quantifying the time spent in the light box by the aforementioned mouse group. Among them, P<0.05 indicates the difference is significant and marked with *; when the p-value is less than 0.01, the difference is extremely significant and marked with **; when the p-value is less than 0.001, the difference is even greater and marked with ***; when the p-value is less than 0.0001, the difference is particularly high and marked with ****; and ns indicates no significant difference.
**Fig. 10** shows the optomotor response of C57BL/6J mice, *rd10* mice treated with intravitreal injection of rAAV2-CMV-VR3.0-EYFP, and untreated littermate *rd10* mice. A: experimental schematic diagram of optomotor response; B: Histogram showing the statistical comparison of visual acuity of the aforementioned mice. Among them, P<0.05 indicates the difference is significant and marked with *; when the p-value is less than 0.01, the difference is extremely significant and marked with **; when the p-value is less than 0.001, the difference is even greater and marked with ***; when the p-value is less than 0.0001, the difference is particularly high and marked with ****; and ns indicates no significant difference.

### DETAIL DESCRIPTION OF THE INVENTION

The following will further illustrate the present application in conjunction with the embodiments. It should be understood that the embodiments are only used to further illustrate and clarify the present application, and are not intended to limit the present application.

Unless otherwise defined, the technical and scientific terms used in this description have the same meanings as those commonly understood by those skilled in the art. Although similar or identical methods and materials can be applied in experiments or practical applications, this application still describes the materials and methods in the following text. In case of conflict, this description, including its definitions, shall prevail. Additionally, materials, methods, and examples are for illustration purposes only and are not restrictive. The following provides further explanation of the present application in conjunction with specific embodiments, but is not intended to limit the scope of the present application.

As used in this article, "light-activated channel protein", "light-activated protein" and "photosensitive protein" can be used interchangeably, referring to a class of proteins on the cell membrane capable of sensing light stimulation and produce specific effects such as changing the open state of ion channels. These proteins can be divided into activating and inhibitory types, which induce neuronal excitation or inhibition.

### light-activated channel protein

The present application provides a light-activated channel protein, comprising a channelrhodopsin (NCR1) comprising the amino acid sequence shown in SEQ ID NO.1 or a variant thereof, or comprising a channelrhodopsin (NCR1) with the amino acid sequence of SEQ ID NO.1 or a variant thereof, wherein the variant is selected from any one of the following:
(1) a protein obtained by truncating 1-23 amino acids at the N-terminus of the channelrhodopsin;
(2) a protein obtained by truncating 90-189 amino acids at the C-terminus of the channelrhodopsin;
(3) a protein obtained by truncating 1-23 amino acids at the N-terminus and 90-189 amino acids at the C-terminus of the channelrhodopsin; or
(4) a protein having at least 60% sequence identity with the protein described in (1), (2), or (3), for example a protein having more than 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 94%, 96%, 98%, 91%, 92%, 93%, 94%, 95%, 96%, 97%, 98%, or 99% sequence identity with the protein described in (1), (2), or (3).

In a specific embodiment, the amino acid sequence of the channelrhodopsin is shown in SEQ ID NO.1.

In a specific embodiment, the variant is a protein obtained by truncating 1-23 amino acids at the N-terminus of channelrhodopsin shown in SEQ ID NO.1. Among them, 1-23 amino acids may be, for example, 1, 2, 3, 4, 5, 6, 7, 8, 9, 10, 11, 12, 13, 14, 15, 16, 17, 18, 19, 20, 21, 22, or 23 amino acids.

In a specific embodiment, the variant is a protein obtained by truncating 5-15 amino acids at the N-terminus of the channelrhodopsin shown in SEQ ID NO.1.

In a specific embodiment, the amino acid sequence of the variant is shown in any one of SEQ ID NO.8-11, or has more than 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 91%, 92% 93%, 94%, 95%, 96%, 97%, 98%, or 99% sequence identity with any one of SEQ ID NO.8-11.

In a specific embodiment, the variant is a protein obtained by truncating 90-189 amino acids at the C-terminus of the channelrhodopsin shown in SEQ ID NO.1. Among them, 90-189 amino acids may be, for example, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136. 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169 170, 171, 172, 173, 174, 175, 176, 177, 178, 179, 180, 181, 182, 183, 184, 185, 186, 187, 188, or 189 amino acids.

In a specific embodiment, the variant is a protein obtained by truncating102-179 amino acids at the C-terminus of channelrhodopsin shown in SEQ ID NO.1.

In a specific embodiment, the amino acid sequence of the variant is shown in any one of SEQ ID NO.12-17, or has more than 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 91%, 92% 93%, 94%, 95%, 96%, 97%, 98%, or 99% sequence identity with any one of SEQ ID NO.12-17.

In a specific embodiment, the variant is a protein obtained by truncating 1-23 amino acids at the N-terminus and 90-189 amino acids at the C-terminus of the channelrhodopsin shown in SEQ ID NO.1. Among them, 1-23 amino acids may be, for example, 1, 2, 3, 4, 5, 6, 7, 8, 9, 10, 11, 12, 13, 14, 15, 16, 17, 18, 19, 20, 21, 22, or 23 amino acids; 90-189 amino acids may be, for example, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136. 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170 171, 172, 173, 174, 175, 176, 177, 178, 179, 180, 181, 182, 183, 184, 185, 186, 187, 188, or 189 amino acids.

In a specific embodiment, the variant is a protein obtained by truncating 5-15 amino acids at the N-terminus and 102-159 amino acids at the C-terminus of the channelrhodopsin shown in SEQ ID NO.1.

In a specific embodiment, the amino acid sequence of the variant is shown in any one of SEQ ID NO.18-26, or has more than 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 91%, 92% 93%, 94%, 95%, 96%, 97%, 98%, or 99% sequence identity with any one of SEQ ID NO.18-26.

In a specific embodiment, the light-activated channel protein further comprises an LR signal peptide fused to the N-terminus of the channelrhodopsin or a variant thereof.

In a specific embodiment, the amino acid sequence of the LR signal peptide is shown in SEQ ID NO.2.

In a specific embodiment, the light-activated channel protein further comprises a T peptide connected to the C-terminus of the channelrhodopsin or a variant thereof to increase cell membrane expression efficiency, and an endoplasmic reticulum export signal sequence E peptide connected to the T peptide.

In a specific embodiment, the amino acid sequence of the T peptide is shown in SEQ ID NO.4, and the amino acid sequence of the E polypeptide is shown in SEQ ID NO.5.

Those skilled in the art can understand that there may also be a linker peptide sequence composed of a small number of amino acids that does not affect the function of the light-activated channel protein between any two of the channelrhodopsin or a variant thereof, T peptide, and E peptide.

In a specific embodiment, the amino acid sequence of the linker peptide is shown in SEQ ID NO.28.

### Nucleic acid molecules, vectors, recombinant viruses

This application provides a nucleic acid molecule comprising a nucleotide sequence encoding any of the aforementioned light-activated channel proteins.

In some embodiments, the nucleic acid molecule is an engineered DNA molecule. In some embodiments, the DNA molecules can replicate and/or be expressed in cells. In some embodiments, the DNA molecule can replicate and/or be expressed in eukaryotic cells. In some embodiments, the DNA molecule may replicate and/or be expressed in prokaryotic cells. In some embodiments, the DNA molecule can be expressed in eukaryotic cells and replicated in prokaryotic cells. Therefore, in addition to the nucleotide sequence encoding the light-activated channel protein, the DNA molecule also comprises genetic manipulation or regulatory elements for replication and/or expression in prokaryotic and/or eukaryotic cells. In some embodiments, the eukaryotic cells are human retinal photoreceptor cells. In some embodiments, the eukaryotic cells are human cone cells. In some embodiments, the eukaryotic cells are bipolar cells or ganglion cells.

This application provides a vector comprising the aforementioned nucleic acid molecule.

In some embodiments, the vector is a DNA plasmid. As used herein, the term "DNA plasmid" refers to a plasmid composed of double stranded DNA molecules. In some embodiments, the "plasmid" is a circular DNA molecule. In some embodiments, the "plasmid" may also encompass linear DNA molecules. Specifically, the term "plasmid" also encompasses molecules obtained by, for example, cleaving a circular plasmid with restriction endonucleases, thereby converting the circular plasmid molecule into a linear form, as well as linear molecules capable of replication in prokaryotes. Plasmids can replicate, i.e., amplify independently of the genomic genetic information stored in the nucleoid of prokaryotic cells, and can be used for cloning, i.e., amplifying genetic information in bacterial cells. For example, the DNA plasmid of the present application is a plasmid constructed based on the pGEMHE plasmid.

This application provides a recombinant virus comprising any of the nucleic acid molecules or vectors mentioned above.

In some embodiments, the recombinant virus is an adeno-associated virus (AAV), chimeric AAV, adenovirus, retrovirus, lentivirus, herpes simplex virus, baculovirus, or any mutant or derivative thereof. Preferably, the recombinant virus is AAV. In some embodiments, the AAV comprises one or more of the following: AAV1, AAV2, AAV3, AAV4, AAV5, AAV6, AAV7, AAV8, AAV9, AAV10, AAV11, AAV12, AAVrh8, AAVrh10, AAVrh36, AAVrh37, AAVrh74, AAVrh79, AAV-DJ, AAV-DJ/8, AAV.Anc80, AAV.Anc80L65, AAV-PHP.B, AAV-PHP.B2, AAV-PHP.B3, AAV-PHP.A, AAV-PHP.eB, AAV-PHP.S, AAV2i8, MyoAAV, AAVMYO, AAV.CPP.16 capsid serotype or a variant thereof.

### Pharmaceutical composition

This application provides a pharmaceutical composition comprising any of the aforementioned light-activated channel proteins, nucleic acid molecules, vectors or recombinant viruses, and a pharmaceutically acceptable carrier.

The form of a pharmaceutical composition depends on multiple criteria, including, for example, route of administration, disease severity, or dosage.

In some embodiments, the pharmaceutical composition may be formulated for delivery to a subject via appropriate routes, including but not limited to oral administration, injection (such as intravenous injection, intramuscular injection, subcutaneous injection, intradermal injection, intracardiac injection, intrathecal injection, intrapleural injection, intraperitoneal injection, etc.), mucosal administration (such as intranasal administration, buccal administration, etc.), sublingual administration, rectal administration, transdermal administration, intraocular administration, or pulmonary administration. According to the intended administration route, the pharmaceutical composition can be prepared as tablet, capsule, pill, sugar-coated pill, powder, granule, cachet, lozenge, suppository, suspension, emulsion, syrup, aerosol (as solid or in liquid medium), spray, ointment, paste, patch, cream, lotion, gel, inhalant, etc.

Those skilled in the art can understand that the dosage and frequency of administration of pharmaceutical composition can vary depending on the age, weight, individual response to vaccines, and the specific administration chosen.

### Treating method and applications

This application provides use of the aforementioned pharmaceutical composition in the preparation of a medicament for treating retinal photoreceptor cell degenerative diseases.

This application provides a method for treating retinal photoreceptor cell degenerative disease, comprising administering a therapeutically effective amount of the above-mentioned pharmaceutical composition to a subject.

Among them, the retinal photoreceptor cell degenerative disease can encompass various known retinal photoreceptor cell degenerative diseases in this field, such as retinitis pigmentosa (RP), age-related macular degeneration (AMD), cone-rod dystrophy, Leber congenital amaurosis (LCA), etc.

It should be understood that this application includes various aspects, embodiments, and combinations of the aspects and/or embodiments described herein. The above description and subsequent examples are intended to illustrate rather than limit the scope of the present application. Other aspects, improvements, and modifications within the scope of this application will be apparent to those skilled in the art to which this application belongs. Therefore, ordinary technical personnel in this field should recognize that the scope of this application also includes the improvements and modifications to the aspects and examples.

### Examples

### Example 1. Construction of expression plasmids of VR3.0 mutants modified based on microbial light-activated protein NCR1

Channelrhodopsin-2 (ChR2) is a membrane protein comprising seven transmembrane helices and a covalently bound retinal chromophore. After ChR2, researchers discovered channelrhodopsins with different ion selectivity. From the genomic data of the cryptophyte alga *H. catenoides,* another type of ChR sequence exhibiting high permeability to Na⁺ ions was identified. This Na⁺-permeable channelrhodopsin was named HcNCR1 (abbreviated as NCR1). The full-length NCR1 protein consists of 432 amino acids. To enhance its photosensitivity in eukaryotic cells, we performed truncation and modifications on NCR1 (432aa) and added several plasma membrane-targeting enhancer peptides at both termini to improve plasma membrane transport efficiency.

Based on our previous research and understanding of the structure **(****Fig. 1****)** and function of the NCR1 light-activated channel protein, we have truncated and modified the amino acid sequence of NCR1 protein (amino acid sequence is shown in SEQ ID NO.1). The NCR1 protein has 7 transmembrane domains, with an extramembrane N-terminal region of approximately more than 20 amino acids and an extramembrane C-terminal region of approximately more than 220 amino acids. The specific truncation and mutation design strategies are shown in **Fig. 2****,** wherein:
there are four N-terminal truncation variants of NCR1, with deletions of 5, 10, 15, and 24 amino acids and designated as NCR1 e1, NCR1 e2, NCR1 e3, and NCR1 e4, respectively;
there are six C-terminal truncation variants of NCR1, with deletions of 102, 126, 149, 159, 179, and 190 amino acids and designated as NCR1 e5, NCR1 e6, NCR1 e7, NCR1 e8, NCR1 e9, and NCR1 e10, respectively;
the N-terminal and C-terminal truncated variants of NCR1 include nine types with the following amino acid deletions: N6-C330 (5 amino acids truncated at the N-terminus and 102 amino acids truncated at the C-terminus), N6-C283 (5 amino acids truncated at the N-terminus and 149 amino acids truncated at the C-terminus), N6-C273 (5 amino acids truncated at the N-terminus and 159 amino acids truncated at the C-terminus), N11-C283 (11 amino acids truncated at the N-terminus and 149 amino acids truncated at the C-terminus), N11-C273 (11 amino acids truncated at the N-terminus and 159 amino acids truncated at the C-terminus), N16-C306 (15 amino acids truncated at the N-terminus and 126 amino acids truncated at the C-terminus), N16-C283 (15 amino acids truncated at the N-terminus and 149 amino acids truncated at the C-terminus), N16-C273 (15 amino acids truncated at the N-terminus and 159 amino acids truncated at the C-terminus), and N16-C242 (15 amino acids truncated at the N-terminus and 190 amino acids truncated at the C-terminus), named NCR1 e11, NCR1 e12, NCR1 e13, NCR1 e14, NCR1 e15, NCR1 e16, NCR1 el7, NCR1 e18, and NCR1 e19, respectively.

We constructed expression plasmids (based on the pGEMHE plasmid backbone) for the aforementioned series of NCR1 light-activated protein variants (NCR1 e1 to e19). An LR signal peptide sequence (amino acid sequence is shown in SEQ ID NO.2) was added to the N-terminus of the light-activated protein. To facilitate detection of the expressed light-activated protein, the following sequences were added to the C-terminus of light-activated protein: (1) a fused fluorescent protein YFP sequence (amino acid sequence is shown in SEQ ID NO.3), (2) a T sequence (amino acid sequence is shown in SEQ ID NO.4) to enhance cell membrane expression efficiency, and (3) an endoplasmic reticulum export signal sequence E (amino acid sequence SEQ ID NO.5). Specifically, the light-activated protein VR3.0 was constructed in the following sequential order: the LR signal peptide sequence at the N-terminus, followed by the amino acid sequence of NCR1 or its variants, and then the T sequence, and finally the E sequence. When fusing with a YFP fluorescent protein tag is required, YFP is inserted between the T sequence and E sequence. When the light-activated protein VR3.0 is used for clinical gene therapy, it does not contain the YFP fluorescent protein.

Wherein the NCR2.0 was obtained by adding an LR signal peptide sequence at the N-terminus, and connecting a T sequence, a YFP sequence, an E sequence, and a linker peptide sequence at the C-terminus of NCR1. NCR2.0 e1-e19 was obtained by adding an LR signal peptide sequence at the N-terminus and connecting a T sequence and an E sequence at the C-terminus of NCR1 e1-e19.

### Example 2. Performance comparison of VR3.0 series variants modified based on microbial light-activated protein NCR1 in Xenopus laevis oocytes

Using the AmpliCap-Max T7 High Yield Message Maker Kit, we synthesized RNA encoding the light-activated channel protein mentioned above, and injected 30 ng each of the aforementioned RNA sample into different Xenopus laevis oocytes. 2 days after injection, photoresponses of *Xenopus laevis* oocytes expressing the light-activated proteins were recorded using a two-electrode voltage clamp. Specifically, electrophysiological measurements of *Xenopus laevis* oocytes were conducted in Ringer's solution (Ori, 110 mM NaCl, 5 mM KCl, 2 mM CaCl₂, 1 mM MgCl₂, 5 mM HEPES, and pH 7.6). The electrode capillary (Φ=1.5 mm, wall thickness 0.178 mm) was filled with 3 M KCl, and an open tip resistance of 0.4-1 MΩ was achieved. Stimulation and data collection were controlled using an AD-DA converter and WinWCP software (v4.1.7). Optical power was measured using a PLUS 2 power and energy meter.

Under illumination irradiation condition (532 nm wavelength, 0.5 mW/mm², -40 mV), photoresponse currents were recorded for the aforementioned light-activated channel protein NCR 2.0 and NCR2.0e series variants (also referred to as VR3.0 series), in comparison with those of the light-activated channel protein NCR1. The results are shown in **Fig. 3****.**

Among them, the current of NCR1 is about 0.41 µA; the current of NCR2.0 is about 6.75 µA, which is about 16.46 times higher than that of NCR1; the current of NCR2.0 e5 is about 13.82 µA, which is about 33.71 times higher than that of NCR1; the current of NCR2.0 e7 is about 17.79 µA, which is about 43.39 times higher than that of NCR1; the current of NCR2.0 e14 is about 27.01 µA, which is about 65.88 times higher than that of NCR1. The experimental results indicate that the NCR2.0 variants, constructed by truncating the N-terminal or (and) C-terminal regions of the NCR1 protein as shown above, exhibited significantly enhanced photoresponse current levels compared to the light-activated channel protein NCR1, and can achieve stronger photosensitivity than the light-activated channel protein NCR1, indicating great potential for applications.

We investigated the trends of photoresponse current changes of VR3.0 series light-activated channel proteins under increasing light intensities. Under 532 nm wavelength illumination, the light intensity was incrementally raised from 0.03 mW/mm² to 4 mW/mm² (specifically: 0.031, 0.062, 0.125, 0.25, 0.5, 1, 2, 3, and 4 mW/mm²). We measured the photocurrents of representative light-activated channel proteins (as shown in **Fig. 4A****).** It was observed that the photoresponse currents of the NCR2.0 series light-activated channel proteins gradually increased with rising light intensity, gradually reaching a plateau at 3 mW/mm². The experimental results show that, under a specific wavelength (532 nm) and within a certain range of light intensities (0.03 mW/mm² to 4 mW/mm²), the tested VR3.0 series light-activated channel proteins exhibited a light-activated channel characteristic where the photoresponse current increases with increasing light intensity.

To test the wavelength range of the photoresponse of the VR3.0 series light-activated channel proteins, the following experiment was conducted. Action spectrum measurements were performed by combining narrow-bandwidth interference filters (Edmund Optics) at different wavelengths with a white light generator PhotoFluor II to cover a range of light wavelengths from 420 nm to 620 nm. The specific wavelengths included were: 422 nm, 439 nm, 459 nm, 481 nm, 496 nm, 516 nm, 540 nm, 562 nm, 595 nm, and 620 nm. To achieve similar light intensities across wavelengths, the output power of the light source was adjusted and gray filters were used to ensure a light intensity of approximately 0.5 mW/mm². The detection results indicated that the NCR2.0 series light-activated channel proteins exhibited significant photocurrent response to wavelengths between 450 nm and 600 nm, with the peak response observed at around 532 nm (as shown in Fig. 4B). Experimental results showed that the VR3.0 series light-activated channel proteins possess a broad continuous wavelength response range, showing photosensitivity across major visible light bands represented by blue, green, and red. Therefore, these *ex vivo* cellular-level photoresponse assays revealed that the engineered VR3.0 series light-activated channel protein variants exhibit significantly improved photoresponse properties compared to natural NCR1 light-activated channel proteins, thereby laying a solid foundation for functional studies and applications at the level of live animals.

### Example 3. Construction of core plasmids expressing light-activated proteins and preparation of rAAVs

We constructed core plasmids containing either the control PsCatCh2.0 light-activated channel protein (amino acid sequence as shown in SEQ ID NO.27) gene or variant genes of the VR3.0 series light-activated channel proteins. These constructs were subsequently packaged into recombinant rAAVs, followed by further validation and functional testing.

To enable efficient expression of the light-activated channel proteins in retinal cells, the broad-spectrum CMV promoter was employed. For convenient detection of the expressed light-activated channel protein, the following genetic elements were incorporated at its C-terminus: (1) a fused YFP sequence, (2) a T sequence to enhance cell membrane expression efficiency, (3) an endoplasmic reticulum export signal sequence E. WPRE elements (nucleotide sequence is shown in SEQ ID NO.6) and HGHpA sequence (nucleotide sequence is shown in SEQ ID NO.7) were also added. The pAAV-MCS plasmid backbone was utilized to construct the pAAV-CMV-VR3.0-T-EYFP-E-WPRE-HGHpA core plasmid series, as shown in Fig. 5. Sequencing results confirmed the correct construction of all plasmids.

We used the commonly used AAV2 serotype and utilized the triple-plasmid transfection method in HEK293 cells to package the rAAV. Specifically, the serotype plasmid pAAV-RC2, the packaging helper plasmid pAAV-Helper, and the core plasmid pAAV-CMV-VR3.0-T-EYFP-E-WPRE-HGHpA were co-transfected into HEK293 cells. After 72 hours, both the cell pellets and the culture supernatant were collected. The desired rAAV particles were subsequently purified via ultracentrifugation using iodixanol (idox), with titer unit of vg/ml. The purified virus was aliquoted and stored at -80°C.

### Example 4. Recording the light response of HEK293T cells expressing VR3.0 series with patch clamp

The core plasmid carrying the expression cassette of the light-activated channel protein VR3.0 series prepared in example 3 was transfected into adherently cultured HEK293T cells. After transfection, the cells were further incubated for 48 hours, followed by whole-cell voltage-clamp recording under constant room temperature (25°C). The main experimental conditions were as follows: the extracellular solution consisted of 140 mM sodium chloride, 5 mM potassium chloride, 2 mM calcium chloride, 20 mM 4-hydroxyethylpiperazine ethanesulfonic acid, and 16 mM glucose, wherein the pH was adjusted to 7.4 with sodium hydroxide, and the solution was placed at room temperature; the intracellular solution consisted of 115 mM cesium methylsulfonate, 20 mM cesium chloride, 2.5 mM magnesium chloride, 0.6 mM ethylene glycol bis (2-aminoethyl ether) tetraacetic acid, 10 mM 4-hydroxyethylpiperazine ethanesulfonic acid, 4 mM adenosine 5'-triphosphate magnesium salt, 0.4 mM guanosine 5'-triphosphate sodium salt, and 10 mM phosphocreatine, wherein the pH was adjusted to 7.2 with cesium hydroxide, and the solution was placed on ice. The extracellular solution was pre-oxygenated with 100% O₂ 30 minutes before the experiment. A P-1000 horizontal puller (Sutter Instrument) was used to pull glass microelectrodes with resistances of 6-8 MΩ. The HEK293T cells to be tested were dark-adapted in the extracellular solution for 30 minutes to stabilize their physiological state before patch-clamp recordings.

To verify the photosensitivity of the target light-activated channel protein, the photocurrents were recorded under various wavelengths and light intensities, and the open-time constant and closed-time constant were analyzed using Clampfit 10.6 software. The results are shown in **Fig. 6****, Table 1,** and **Fig. 7****.**

**Table 1**

| **Name of light-activated channel protein** | **PsCat Ch2.0** | **NCR 2.0** | **NCR 2.0-e 1** | **NCR 2.0-e 2** | **NCR 2.0-e 3** | **NCR 2.0-e 4** | **NCR 2.0-e 5** | **NCR 2.0-e 6** | **NCR 2.0-e 7** | **NCR 2.0-e 8** | **NCR 2.0-e 9** |
|---|---|---|---|---|---|---|---|---|---|---|---|
| Average photocurrent value at 470 nm (pA), light intensity 1.7×10¹⁵ | 476.67 | 65.00 | 200.0 0 | 685.0 0 | 550.0 0 | 0.00 | 127.5 0 | 1190. 00 | 1710. 00 | 432.5 0 | 112.5 0 |
| photons/cm²s | | | | | | | | | | | |
| Average photocurrent value at 530 nm (pA), light intensity 1.9×10¹⁵ photons/cm²s | 22.50 | 97.00 | 360.0 0 | 890.0 0 | 492.5 0 | 0.00 | 200.0 0 | 1480. 00 | 1703. 33 | 310.0 0 | 127.5 0 |
| Average photocurrent value at 590 nm (pA), light intensity 2.1×10¹⁵ photons/cm² s | 0.00 | 82.00 | 130.0 0 | 667.5 0 | 690.0 0 | 0.00 | 130.0 0 | 922.5 0 | 1706. 67 | 360.0 0 | 60.00 |

| **Name of light-activated channel protein** | **PsCat Ch2.0** | **NCR 2.0-e 10** | **NCR 2.0-e 11** | **NCR 2.0-e 12** | **NCR 2.0-e 13** | **NCR 2.0-e 14** | **NCR 2.0-e 15** | **NCR 2.0-e 16** | **NCR 2.0-e 17** | **NCR 2.0-e 18** | **NCR 2.0-e 19** |
|---|---|---|---|---|---|---|---|---|---|---|---|
| Average photocurrent value at 470 nm (pA), light intensity 1.7×10¹⁵ photons/cm²s | 476.67 | 0.00 | 307.5 0 | 1840. 00 | 982.9 7 | 1005. 00 | 720.0 0 | 670.0 0 | 816.2 5 | 905.0 0 | 0.00 |
| Average photocurrent value at 530 nm (pA), light intensity 1.9×10¹⁵ photons/cm²s | 22.50 | 0.00 | 380.0 0 | 1722. 00 | 965.0 0 | 905.0 0 | 785.0 0 | 820.0 0 | 947.5 0 | 946.2 5 | 0.00 |
| Average photocurrent value at 590 nm (pA), light intensity 2.1×10¹⁵ photons/cm²s | 0.00 | 0.00 | 182.5 0 | 1538. 33 | 850.0 0 | 728.7 5 | 685.0 0 | 570.0 0 | 771.2 5 | 797.5 0 | 0.00 |

As illustrated in **Fig. 6** and **Table 1,** the photoresponse current levels of the VR3.0 series variants under different wavelengths and light intensities are presented. The results show that the control group PsCatCh2.0 exhibited photoresponses only to 470 nm wavelength, with almost no photoresponse to 530 nm and 590 nm. From **Table 1,** it is demontrated that compared to the control group PsCatCh2.0, the VR3.0 series variants in the experimental group also have better photosensitivity to 530 nm and 590 nm light stimuli, indicating a broader wavelength range of photoresponse. Therefore, under natural light conditions, the VR3.0 series variants of this application exhibit a significant advantage in terms of photoresponse sensitivity.

According to the guidelines established by the International Commission on Non Ionizing Radiation Protection (ICIRP), the light intensity applied to the retina must not exceed the safety threshold for the corresponding wavelengths. The maximum permissible light intensity for blue light at 470 nm is 7.6×10¹⁴ photons/cm²s, for green light at 530 nm is 2.5×10¹⁶ photons/cm²s, and for red light at 590 nm is 5.9× 10¹⁷photons/cm²s. As shown in **Fig. 7A****,** experimental results indicate that most variants of the light-activated channel protein VR3.0 series can elicit photocurrents at light intensities of 6.9×10¹² photons/cm²s (wavelength 470 nm), 6.7×10¹³ photons/cm²s (wavelength 530 nm), and 7.4×10¹³ photons/cm²s (wavelength 590 nm), all of which can generate photocurrent below the safe light intensity threshold of the retina without causing phototoxic effects on the retina.

As shown in **Fig. 7A** and **7B****,** the photoresponse current amplitudes of most variants in the light-activated channel protein VR3.0 series also exhibited an increasing trend with rising light intensity, demonstrating a notable improvement compared to the control light-activated channel protein PsCatCh2.0. Under the condition of 470 nm wavelength and a light intensity of 1.7×10¹⁵ photons/cm²s, the photocurrent amplitudes of NCR2.0 e2, NCR2.0 e3, NCR2.0 e6, NCR2.0 e7, NCR2.0 e12, NCR2.0 e13, NCR2.0 e14, NCR2.0 e16, NCR2.0 e17, and NCR2.0 e18 were all higher than that of PsCatCh2.0.

Visual restoration requires high spatiotemporal resolution, which requires light-activated channel proteins to have fast kinetics characteristics with high spatiotemporal resolution. Therefore, we also detected the light-response frequency of the light-activated channel proteins by applying pulsed light stimuli at 2 Hz, 4 Hz, 8 Hz, 16 Hz, and 32 Hz. Light was delivered via a Mightex external fiber optical fiber, with stimulation timing controlled by the BioLED software, and the specific light intensity was measured using an optical power meter. Under 470 nm wavelength illumination with an intensity of 1.7×10¹⁵ photons/cm²s, a 1-second stimulation elicited current responses, as shown in **Fig. 8****.** The results show that visual signal processing requires a response frequency of 24 Hz, and some variants of the light-activated channel protein VR3.0 series were capable of responding to 32 Hz light stimuli, thereby meeting the requirements of visual signals.

### Example 5. Intravitreal injection of rAAVs in rd10 mice

For the animal experiments, postnatal 4-week-old retinal pigment degeneration model mice (*rd10* mice) and wild-type C57BL/6J mice were selected. The mice were anesthetized via intraperitoneal injection with a mixture of 100 mg/kg ketamine and 12 mg/kg xylazine based on body weight. After achieving sufficient anesthesia, the ocular surface and periorbital skin were disinfected with 0.5% povidone-iodine. To minimize discomfort during intravitreal injection, topical anesthesia was administered to the mouse eyes using proparacaine hydrochloride eye drops (Alcaine). The mice were immobilized and the eyeballs were exposed. 1.5 µL of rAAV2-CMV-VR3.0 virus (titer: approximately 5.0×10¹² vg/mL) was taken using a Nanoject III high-precision microinjector equipped with a glass microelectrode. The injection was performed intravitreally by inserting the needle 0.5 mm below the corneal limbus on the nasal side of the mouse, and the contralateral eye was injected in the same manner. A control group receiving only the injection vehicle was established in parallel. After the injection, levofloxacin hydrochloride ophthalmic gel (Jie Qi) was applied to the mouse eyeballs to prevent infection. One month post-injection, therapeutic efficacy assessment and behavioral observation were conducted in *rd10* mice.

### Example 6. Light induced light/dark box behavior experiment in mice

The light/dark box consists of two equally sized compartments (18 cm × 20 cm × 18 cm) on both sides, connected by an arched doorway (7 cm × 5 cm). The light box was equipped with a LED light source (Mightex, Canada), while the dark box was wrapped with a black cloth cover. All experimental mice were aged 10-12 weeks and dark-adapted for 2 hours before the experiment. All behavioral experiments were conducted between 18:00 and 21:00. At the beginning of the experiment, C57BL/6J mice, *rd10* mice treated with intravitreal injection of rAAV2-CMV-VR3.0, and untreated *rd10* mice were individually placed in the bright box with a central white light intensity of 190 lux (Lux), and allowed to explore freely. Mouse head position was analyzed to determine their movement patterns between light and dark boxes. Subsequently, the collected data was imported into GraphPad Prism 7 software and evaluated for significance using one-way ANOVA, with P<0.05 indicating statistically significant.

We analyzed and compared the proportion of activity time of mice in the light box relative to the total time, and the experimental results are shown in **Fig. 9****.** The results showed that the wild-type C57BL/6J mice injected with solvent (positive control) exhibited a value of 13.42%, n=9; while the *rd10* mice injected with solvent (negative control) showed a value of 68.78%, n=9; *rd10* mice treated with rAAV2-CMV-NCR2.0 e14 virus injection displayed a value of 36.14%, n=9. These findings indicate that intravitreal injection of rAAV2-CMV-VR3.0 restored the light-avoidance behavior in *rd10* mice, proving that the VR3.0 series light-activated channel proteins (such as NCR2.0 e14) can restore visually guided behavior in *rd10* mice with retinal degeneration and their efficacy in treating retinal degeneration.

### Example 7. Mouse optomotor response behavior experiment

4 Lenovo monitors (L1900pA) were used to display moving gratings, with a central activity platform (height: 17.5 cm) for mice, and a mirror surface on the bottom. the grating parameters were programmed using Matlab, with each experiment lasting 12 minutes. In each phase, the grating rotated clockwise for 30 seconds, counterclockwise for 30 seconds, followed by a 10 s pause. The angular velocity of the grating rotation was 12 °/s. The spatial frequency of the gratings at each stage (i.e., the number of gratings bars within 1°, in cycles/degree (c/d)) was sequentially set as follows: 0.20 (for software and system testing), 0.10, 0.15, 0.20, 0.25, 0.30, 0.35, 0.40, 0.45, 0.50, and 0.60 c/d. Before the experiment, the mice were dark-adapted for 12 hours. C57BL/6J mice, *rd10* mice treated with intravitreal injection of rAAV2-CMV-VR3.0, and *rd10* mice were individually placed on an activity platform, and the visual acuity was evaluated using the optomotor system. Subsequently, the collected data was imported into GraphPad Prism 7 software to generate bar graphs. Statistical significance was evaluated using Student's t-test, with P<0.05 indicating statistically significant.

The experimental results are shown in **Fig. 10****.** The results indicate that the average maximum visual acuity of wild-type C57BL/6J mice injected with solvent (positive control) was 0.49 c/d, n=7; the average maximum visual acuity of the *rd10* mice injection solvent (negative control) was 0.08 c/d, n=7; and the maximum visual acuity of *rd10* mice treated with injection of rAAV2-CMV-NCR2.0 e14 virus was 0.28 c/d, n=8. It can be seen that intravitreal injection of rAAV2-CMV-VR3.0 significantly restored light sensitivity in *rd10* mice, demonstrating that VR3.0-series light-activated channel proteins (such as NCR2.0 e14) can restore visual-guided behavior in *rd10* mice with retinal degeneration and is effective in treating retinal degeneration.

The sequences involved in the above examples are shown in **Table 2.**

**Table 2: listing of related sequences**

| Sequence number | Sequence description | Sequence |
|---|---|---|
| SEQ ID NO.1 | NCR1 sequence | MPFCGGRPEDGWHHGSIHDMDYPLLGAMAAICSVFIGGSGAWMLYRLDLG LGYSCKPHHSGYAPEANSFSALSCLVSGTIYAAKTFDFFDGGGTPFSFNWYW YLDYVFTCPLILLDVLYTLEIPHKLRFVFAVIITLWCGVAAFVTPSAFRFGYYA VGCVWFVPFSFSLLRHVKQRYQVYPPKCQKLLFWACTIFFGFWPLFPILFLFS WLGTGHIDQQAFTIIHAFLDLFCKTVFGLIMTFFRLELEEHTEVLGLPLNEPK GKHLFLNDSAARHEKHLAGPGTESLTAASSKVKLQWIQKLLAEALTEASQL DLDTPTVVPAKVLSSATPERHLPLRQQKKEQQERGEKTMVYPQLSSAILLGS PAQEEEQGGGGGAKRERGRALVTPLPGDESPGGEHLPDSVQEHLLLAVGSH RMGQQNLRQLRERRRH |
| SEQ ID NO.2 | LR signal peptide sequence | MRPQILLLLALLTLGLANGTEGPNFYVPFSNKTGVVRS |
| SEQ ID NO.3 | Fluorescent protein YFP sequence | MVSKGEELFTGVVPILVELDGDVNGHKFSVSGEGEGDATYGKLTLKFICTTG KLPVPWPTLVTTFGYGLQCFARYPDHMKQHDFFKSAMPEGYVQERTIFFKD DGNYKTRAEVKFEGDTLVNRIELKGIDFKEDGNILGHKLEYNYNSHNVYIM ADKQKNGIKVNFKIRHNIEDGSVQLADHYQQNTPIGDGPVLLPDNHYLSYQ SALSKDPNEKRDHMVLLEFVTAAGITLGMDELYK |
| SEQ ID NO.4 | T peptide sequence | KSRITSEGEYIPLDQIDINV |
| SEQ ID NO.5 | E peptide sequence | FCYENEV |
| SEQ ID NO.6 | WPRE sequence | AATCAACCTCTGGATTACAAAATTTGTGAAAGATTGACTGGTATTCTTAAC TATGTTGCTCCTTTTACGCTATGTGGATACGCTGCTTTAATGCCTTTGTATCA TGCTATTGCTTCCCGTATGGCTTTCATTTTCTCCTCCTTGTATAAATCCTGGT TGCTGTCTCTTTATGAGGAGTTGTGGCCCGTTGTCAGGCAACGTGGCGTG GTGTGCACTGTGTTTGCTGACGCAACCCCCACTGGTTGGGGCATTGCCAC CACCTGTCAGCTCCTTTCCGGGACTTTCGCTTTCCCCCTCCCTATTGCCAC GGCGGAACTCATCGCCGCCTGCCTTGCCCGCTGCTGGACAGGGGCTCGGC TGTTGGGCACTGACAATTCCGTGGTGTTGTCGGGGAAATCATCGTCCTTTC CTTGGCTGCTCGCCTATGTTGCCACCTGGATTCTGCGCGGGACGTCCTTCT GCTACGTCCCTTCGGCCCTCAATCCAGCGGACCTTCCTTCCCGCGGCCTGC TGCCGGCTCTGCGGCCTCTTCCGCGTCTTCGCCTTCGCCCTCAGACGAGT CGGATCTCCCTTTGGGCCGCCTCCCCGC |
| SEQ ID NO.7 | HGH pA sequence | GGGTGGCATCCCTGTGACCCCTCCCCAGTGCCTCTCCTGGCCCTGGAAGT TGCCACTCCAGTGCCCACCAGCCTTGTCCTAATAAAATTAAGTTGCATCAT TTTGTCTGACTAGGTGTCCTTCTATAATATTATGGGGTGGAGGGGGGTGGT ATGGAGCAAGGGGCAAGTTGGGAAGACAACCTGTAGGGCCTGCGGGGTC TATTGGGAACCAAGCTGGAGTGCAGTGGCACAATCTTGGCTCACTGCAAT CTCCGCCTCCTGGGTTCAAGCGATTCTCCTGCCTCAGCCTCCCGAGTTGTT |
| | | GGGATTCCAGGCATGCATGACCAGGCTCAGCTAATTTTTGTTTTTTTGGTA GAGACGGGGTTTCACCATATTGGCCAGGCTGGTCTCCAACTCCTAATCTCA GGTGATCTACCCACCTTGGCCTCCCAAATTGCTGGGATTACAGGCGTGAA CCACTGCTCCCTTCCCTGTCCTT |
| SEQ ID NO.8 | NCR2.0 e1 sequence | GRPEDGWHHGSIHDMDYPLLGAMAAICSVFIGGSGAWMLYRLDLGLGYSC KPHHSGYAPEANSFSALSCLVSGTIYAAKTFDFFDGGGTPFSFNWYWYLDYV FTCPLILLDVLYTLEIPHKLRFVFAVIITLWCGVAAFVTPSAFRFGYYAVGCVW FVPFSFSLLRHVKQRYQVYPPKCQKLLFWACTIFFGFWPLFPILFLFSWLGTG HIDQQAFTIIHAFLDLFCKTVFGLIMTFFRLELEEHTEVLGLPLNEPKGKHLFL NDSAARHEKHLAGPGTESLTAASSKVKLQWIQKLLAEALTEASQLDLDTPTV VPAKVLSSATPERHLPLRQQKKEQQERGEKTMVYPQLSSAILLGSPAQEEEQ GGGGGAKRERGRALVTPLPGDESPGGEHLPDSVQEHLLLAVGSHRMGQQNL RQLRERRRH |
| SEQ ID NO.9 | NCR2.0 e2 sequence | GWHHGSIHDMDYPLLGAMAAICSVFIGGSGAWMLYRLDLGLGYSCKPHHS GYAPEANSFSALSCLVSGTIYAAKTFDFFDGGGTPFSFNWYWYLDYVFTCPLI LLDVLYTLEIPHKLRFVFAVIITLWCGVAAFVTPSAFRFGYYAVGCVWFVPFSF SLLRHVKQRYQVYPPKCQKLLFWACTIFFGFWPLFPILFLFSWLGTGHIDQQ AFTIIHAFLDLFCKTVFGLIMTFFRLELEEHTEVLGLPLNEPKGKHLFLNDSA ARHEKHLAGPGTESLTAASSKVKLQWIQKLLAEALTEASQLDLDTPTVVPAK VLSSATPERHLPLRQQKKEQQERGEKTMVYPQLSSAILLGSPAQEEEQGGGG GAKRERGRALVTPLPGDESPGGEHLPDSVQEHLLLAVGSHRMGQQNLRQLR ERRRH |
| SEQ ID NO.10 | NCR2.0 e3 sequence | SIHDMDYPLLGAMAAICSVFIGGSGAWMLYRLDLGLGYSCKPHHSGYAPEA NSFSALSCLVSGTIYAAKTFDFFDGGGTPFSFNWYWYLDYVFTCPLILLDVLY TLEIPHKLRFVFAVIITLWCGVAAFVTPSAFRFGYYAVGCVWFVPFSFSLLRHV KQRYQVYPPKCQKLLFWACTIFFGFWPLFPILFLFSWLGTGHIDQQAFTIIHAF LDLFCKTVFGLIMTFFRLELEEHTEVLGLPLNEPKGKHLFLNDSAARHEKHL AGPGTESLTAASSKVKLQWIQKLLAEALTEASQLDLDTPTVVPAKVLSSATPE RHLPLRQQKKEQQERGEKTMVYPQLSSAILLGSPAQEEEQGGGGGAKRERG RALVTPLPGDESPGGEHLPDSVQEHLLLAVGSHRMGQQNLRQLRERRRH |
| SEQ ID NO.11 | NCR2.0 e4 sequence | LGAMAAICSVFIGGSGAWMLYRLDLGLGYSCKPHHSGYAPEANSFSALSCLV SGTIYAAKTFDFFDGGGTPFSFNWYWYLDYVFTCPLILLDVLYTLEIPHKLRF VFAVIITLWCGVAAFVTPSAFRFGYYAVGCVWFVPFSFSLLRHVKQRYQVYPP KCQKLLFWACTIFFGFWPLFPILFLFSWLGTGHIDQQAFTIIHAFLDLFCKTVF GLIMTFFRLELEEHTEVLGLPLNEPKGKHLFLNDSAARHEKHLAGPGTESLT AASSKVKLQWIQKLLAEALTEASQLDLDTPTVVPAKVLSSATPERHLPLRQQ KKEQQERGEKTMVYPQLSSAILLGSPAQEEEQGGGGGAKRERGRALVTPLP GDESPGGEHLPDSVQEHLLLAVGSHRMGQQNLRQLRERRRH |
| SEQ ID NO.12 | NCR2.0 e5 sequence | MPFCGGRPEDGWHHGSIHDMDYPLLGAMAAICSVFIGGSGAWMLYRLDLG LGYSCKPHHSGYAPEANSFSALSCLVSGTIYAAKTFDFFDGGGTPFSFNWYW YLDYVFTCPLILLDVLYTLEIPHKLRFVFAVIITLWCGVAAFVTPSAFRFGYYA VGCVWFVPFSFSLLRHVKQRYQVYPPKCQKLLFWACTIFFGFWPLFPILFLFS WLGTGHIDQQAFTIIHAFLDLFCKTVFGLIMTFFRLELEEHTEVLGLPLNEPK GKHLFLNDSAARHEKHLAGPGTESLTAASSKVKLQWIQKLLAEALTEASQL DLDTPTVVPAKVLSSAT |
| SEQ ID NO.13 | NCR2.0 e6 sequence | MPFCGGRPEDGWHHGSIHDMDYPLLGAMAAICSVFIGGSGAWMLYRLDLG LGYSCKPHHSGYAPEANSFSALSCLVSGTIYAAKTFDFFDGGGTPFSFNWYW YLDYVFTCPLILLDVLYTLEIPHKLRFVFAVIITLWCGVAAFVTPSAFRFGYYA VGCVWFVPFSFSLLRHVKQRYQVYPPKCQKLLFWACTIFFGFWPLFPILFLFS WLGTGHIDQQAFTIIHAFLDLFCKTVFGLIMTFFRLELEEHTEVLGLPLNEPK GKHLFLNDSAARHEKHLAGPGTESLTAASSKVKLQWIQKLLAEA |
| SEQ ID NO.14 | NCR2.0 e7 sequence | MPFCGGRPEDGWHHGSIHDMDYPLLGAMAAICSVFIGGSGAWMLYRLDLG LGYSCKPHHSGYAPEANSFSALSCLVSGTIYAAKTFDFFDGGGTPFSFNWYW YLDYVFTCPLILLDVLYTLEIPHKLRFVFAVIITLWCGVAAFVTPSAFRFGYYA VGCVWFVPFSFSLLRHVKQRYQVYPPKCQKLLFWACTIFFGFWPLFPILFLFS WLGTGHIDQQAFTIIHAFLDLFCKTVFGLIMTFFRLELEEHTEVLGLPLNEPK GKHLFLNDSAARHEKHLAGPG |
| SEQ ID NO.15 | NCR2.0 e8 sequence | MPFCGGRPEDGWHHGSIHDMDYPLLGAMAAICSVFIGGSGAWMLYRLDLG LGYSCKPHHSGYAPEANSFSALSCLVSGTIYAAKTFDFFDGGGTPFSFNWYW YLDYVFTCPLILLDVLYTLEIPHKLRFVFAVIITLWCGVAAFVTPSAFRFGYYA VGCVWFVPFSFSLLRHVKQRYQVYPPKCQKLLFWACTIFFGFWPLFPILFLFS WLGTGHIDQQAFTIIHAFLDLFCKTVFGLIMTFFRLELEEHTEVLGLPLNEPK GKHLFLNDSAA |
| SEQ ID NO.16 | NCR2.0 e9 sequence | MPFCGGRPEDGWHHGSIHDMDYPLLGAMAAICSVFIGGSGAWMLYRLDLG LGYSCKPHHSGYAPEANSFSALSCLVSGTIYAAKTFDFFDGGGTPFSFNWYW YLDYVFTCPLILLDVLYTLEIPHKLRFVFAVIITLWCGVAAFVTPSAFRFGYYA VGCVWFVPFSFSLLRHVKQRYQVYPPKCQKLLFWACTIFFGFWPLFPILFLFS WLGTGHIDQQAFTIIHAFLDLFCKTVFGLIMTFFRLELEEHTEV |
| SEQ ID NO.17 | NCR2.0 e10 sequence | MPFCGGRPEDGWHHGSIHDMDYPLLGAMAAICSVFIGGSGAWMLYRLDLG LGYSCKPHHSGYAPEANSFSALSCLVSGTIYAAKTFDFFDGGGTPFSFNWYW YLDYVFTCPLILLDVLYTLEIPHKLRFVFAVIITLWCGVAAFVTPSAFRFGYYA VGCVWFVPFSFSLLRHVKQRYQVYPPKCQKLLFWACTIFFGFWPLFPILFLFS WLGTGHIDQQAFTIIHAFLDLFCKTVFGLIMTF |
| SEQ ID NO.18 | NCR2.0 ell sequence | GRPEDGWHHGSIHDMDYPLLGAMAAICSVFIGGSGAWMLYRLDLGLGYSC KPIIIISGYAPEANSFSALSCLVSGTIYAAKTFDFFDGGGTPFSFNWYWYLDYV FTCPLILLDVLYTLEIPHKLRFVFAVIITLWCGVAAFVTPSAFRFGYYAVGCVW FVPFSFSLLRHVKQRYQVYPPKCQKLLFWACTIFFGFWPLFPILFLFSWLGTG HIDQQAFTIIHAFLDLFCKTVFGLIMTFFRLELEEHTEVLGLPLNEPKGKHLFL NDSAARHEKHLAGPGTESLTAASSKVKLQWIQKLLAEALTEASQLDLDTPTV VPAKVLSSAT |
| SEQ ID NO.19 | NCR2.0 e12 sequence | GRPEDGWHHGSIHDMDYPLLGAMAAICSVFIGGSGAWMLYRLDLGLGYSC KPHHSGYAPEANSFSALSCLVSGTIYAAKTFDFFDGGGTPFSFNWYWYLDYV FTCPLILLDVLYTLEIPHKLRFVFAVIITLWCGVAAFVTPSAFRFGYYAVGCVW FVPFSFSLLRHVKQRYQVYPPKCQKLLFWACTIFFGFWPLFPILFLFSWLGTG HIDQQAFTIIHAFLDLFCKTVFGLIMTFFRLELEEHTEVLGLPLNEPKGKHLFL NDSAARHEKHLAGPG |
| SEQ ID NO.20 | NCR2.0 e13 sequence | GRPEDGWHHGSIHDMDYPLLGAMAAICSVFIGGSGAWMLYRLDLGLGYSC KPHHSGYAPEANSFSALSCLVSGTIYAAKTFDFFDGGTPFSFNWYWYLDYV FTCPLILLDVLYTLEIPHKLRFVFAVIITLWCGVAAFVTPSAFRFGYYAVGCVW FVPFSFSLLRHVKQRYQVYPPKCQKLLFWACTIFFGFWPLFPILFLFSWLGTG |
| | | HIDQQAFTIIHAFLDLFCKTVFGLIMTFFRLELEEHTEVLGLPLNEPKGKHLFL NDSAA |
| SEQ ID NO.21 | NCR2.0 e14 sequence | GWHHGSIHDMDYPLLGAMAAICSVFIGGSGAWMLYRLDLGLGYSCKPHHS GYAPEANSFSALSCLVSGTIYAAKTFDFFDGGGTPFSFNWYWYLDYVFTCPLI LLDVLYTLEIPHKLRFVFAVIITLWCGVAAFVTPSAFRFGYYAVGCVWFVPFSF SLLRHVKQRYQVYPPKCQKLLFWACTIFFGFWPLFPILFLFSWLGTGHIDQQ AFTIIHAFLDLFCKTVFGLIMTFFRLELEEHTEVLGLPLNEPKGKHLFLNDSA ARHEKHLAGPG |
| SEQ ID NO.22 | NCR2.0 el5 sequence | GWHHGSIHDMDYPLLGAMAAICSVFIGGSGAWMLYRLDLGLGYSCKPHHS GYAPEANSFSALSCLVSGTIYAAKTFDFFDGGGTPFSFNWYWYLDYVFTCPLI LLDVLYTLEIPHKLRFVFAVIITLWCGVAAFVTPSAFRFGYYAVGCVWFVPFSF SLLRHVKQRYQVYPPKCQKLLFWACTIFFGFWPLFPILFLFSWLGTGHIDQQ AFTIIHAFLDLFCKTVFGLIMTFFRLELEEHTEVLGLPLNEPKGKHLFLNDSA A |
| SEQ ID NO.23 | NCR2.0 e16 sequence | SIHDMDYPLLGAMAAICSVFIGGSGAWMLYRLDLGLGYSCKPHHSGYAPEA NSFSALSCLVSGTIYAAKTFDFFDGGGTPFSFNWYWYLDYVFTCPLILLDVLY TLEIPHKLRFVFAVIITLWCGVAAFVTPSAFRFGYYAVGCVWFVPFSFSLLRHV KQRYQVYPPKCQKLLFWACTIFFGFWPLFPILFLFSWLGTGHIDQQAFTIIHAF LDLFCKTVFGLIMTFFRLELEEHTEVLGLPLNEPKGKHLFLNDSAARHEKHL AGPGTESLTAASSKVKLQWIQKLLAEA |
| SEQ ID NO.24 | NCR2.0 e17 sequence | SIHDMDYPLLGAMAAICSVFIGGSGAWMLYRLDLGLGYSCKPHHSGYAPEA NSFSALSCLVSGTIYAAKTFDFFDGGGTPFSFNWYWYLDYVFTCPLILLDVLY TLEIPHKLRFVFAVIITLWCGVAAFVTPSAFRFGYYAVGCVWFVPFSFSLLRHV KQRYQVYPPKCQKLLFWACTIFFGFWPLFPILFLFSWLGTGHIDQQAFTIIHAF LDLFCKTVFGLIMTFFRLELEEHTEVLGLPLNEPKGKHLFLNDSAARHEKHL AGPG |
| SEQ ID NO.25 | NCR2.0 el8 sequence | SIHDMDYPLLGAMAAICSVFIGGSGAWMLYRLDLGLGYSCKPHHSGYAPEA NSFSALSCLVSGTIYAAKTFDFFDGGGTPFSFNWYWYLDYVFTCPLILLDVLY TLEIPHKLRFVFAVIITLWCGVAAFVTPSAFRFGYYAVGCVWFVPFSFSLLRHV KQRYQVYPPKCQKLLFWACTIFFGFWPLFPILFLFSWLGTGHIDQQAFTIIHAF LDLFCKTVFGLIMTFFRLELEEHTEVLGLPLNEPKGKHLFLNDSAA |
| SEQ ID NO.26 | NCR2.0 el9 sequence | SIHDMDYPLLGAMAAICSVFIGGSGAWMLYRLDLGLGYSCKPHHSGYAPEA NSFSALSCLVSGTIYAAKTFDFFDGGGTPFSFNWYWYLDYVFTCPLILLDVLY TLEIPHKLRFVFAVIITLWCGVAAFVTPSAFRFGYYAVGCVWFVPFSFSLLRHV KQRYQVYPPKCQKLLFWACTIFFGFWPLFPILFLFSWLGTGHIDQQAFTIIHAF LDLFCKTVFGLIMTF |
| SEQ ID NO.27 | PsCatCh2.0 sequence | MGFQLNPEYLNETILLDDCTPIYLNVGPLWEQKVARGTQWFGVILSLAFLIY YIWITYKATCGWEELYVCTIEFCKIVIELYFEFSPPAMIYQTNGEVTPWLRYAE WLLTCPVICIHLSNITGLNDDYSGRTMSLITSDLGGICMAVTSALSKGWLKW LFFVIGCCYGASTFYHAALIYIESYYTMPHGVCKNMVLAMAAVFFTSWFMF PGLFLAGPEGTNALSWAGSTIGHTVADLLSKNAWGMIGHFLRLEIHKHIIIHG DVRRPITVNTLGREVTVSCFVDKEEEDEDERISTKTYANRALE |
| SEQ ID | Linker | VDTSSRSR |
| NO.28 | peptide sequence | |

Among them, the amino acid sequences of the aforementioned NCR2.0 e1-e19 only include the NCR1 variant part, i.e., NCR1 e1-e19, and do not include the LR signal peptide, T peptide, and E peptide parts. The amino acid sequence of PsCatCh2.0 also does not include the LR signal peptide, T peptide, and E peptide.

## Claims

1. A light-activated channel protein comprising a channelrhodopsin comprising an amino acid sequence shown in SEQ ID NO.1 or a variant thereof, wherein the variant is selected from any one of the following:
(1) a protein obtained by truncating 1-23 amino acids at the N-terminus of the channelrhodopsin;
(2) a protein obtained by truncating 90-189 amino acids at the C-terminus of the channelrhodopsin;
(3) a protein obtained by truncating 1-23 amino acids at the N-terminus and 90-189 amino acids at the C-terminus of the channelrhodopsin; or
(4) a protein having more than 90%, 91%, 92%, 93%, 94%, 95%, 96%, 97%, 98%, or 99% sequence identity with the protein described in (1), (2), or (3).

2. The light-activated channel protein according to claim 1, wherein the amino acid sequence of the channelrhodopsin is shown in SEQ ID NO.1.

3. The light-activated channel protein according to claim 1 or 2, wherein the variant is a protein obtained by truncating 5-15 amino acids at the N-terminus of the channelrhodopsin.

4. The light-activated channel protein according to claim 1 or 2, wherein the variant is a protein obtained by truncating 102-179 amino acids at the C-terminus of the channelrhodopsin.

5. The light-activated channel protein according to claim 1 or 2, wherein the variant is a protein obtained by truncating 5-15 amino acids at the N-terminus and 102-179 amino acids at the C-terminus of the channelrhodopsin.

6. The light-activated channel protein according to any one of claims 1-5, wherein the amino acid sequence of the variant is shown in any one of SEQ ID NO.8-26, or
has more than 90%, 91%, 92%, 93%, 94%, 95%, 96%, 97%, 98%, or 99% sequence identity with any one of SEQ ID NO.8-26.

7. The light-activated channel protein according to any one of claims 1-6, wherein the light-activated channel protein further comprises an LR signal peptide fused to the N-terminus of the channelrhodopsin or a variant thereof,
and/or a T peptide linked to the C-terminus of the channelrhodopsin or a variant thereof to enhance cell membrane expression efficiency, and an endoplasmic reticulum output signal sequence E peptide linked to the T peptide.

8. The light-activated channel protein according to claim 7, wherein the amino acid sequence of the LR signal peptide is shown in SEQ ID NO.2, the amino acid sequence of the T peptide is shown in SEQ ID NO.4, and the amino acid sequence of the E peptide is shown in SEQ ID NO.5.

9. The light-activated channel protein according to claim 7 or 8, wherein a linker peptide sequence is further included between any two of the channelrhodopsin or a variant thereof, the T peptide, and the E peptide.

10. The light-activated channel protein according to claim 9, wherein the amino acid sequence of the linker peptide is shown in SEQ ID NO.28.

11. A nucleic acid molecule comprising a nucleotide sequence encoding the light-activated channel protein according to any one of claims 1-10.

12. A vector comprising the nucleic acid molecule according to claim 11.

13. A recombinant virus comprising the nucleic acid molecule according to claim 11 or the vector according to claim 12.

14. The recombinant virus according to claim 13, wherein the recombinant virus is a recombinant adeno-associated virus.

15. A pharmaceutical composition comprising the light-activated channel protein according to any one of claims 1-10, the nucleic acid molecule according to claim 11, the vector according to claim 12, or the recombinant virus according to claim 13 or 14, and a pharmaceutically acceptable carrier.

16. Use of the pharmaceutical composition according to claim 15 in the preparation of a medicament for treating retinal photoreceptor cell degenerative diseases.

17. The use according to claim 16, wherein the retinal photoreceptor cell degenerative disease comprises retinitis pigmentosa (RP), age-related macular degeneration (AMD), cone-rod dystrophy, and Leber congenital amaurosis (LCA).

18. A method for treating retinal photoreceptor cell degenerative diseases, comprising administering a therapeutic effective amount of the pharmaceutical composition according to claim 15 to a subject.

19. The method according to claim 18, wherein the retinal photoreceptor cell degenerative disease comprises retinitis pigmentosa (RP), age-related macular degeneration (AMD), cone-rod dystrophy, and Leber congenital amaurosis (LCA).
